# FASCICULE DE BREVET EUROPEEN

(11) **EP 1 737 963 B1**
(45) Date de publication et mention de la délivrance du brevet: **11.05.2016**
(21) Numéro de dépôt: 05762398.5
(22) Date de dépôt: 22.04.2005
(51) Int. Cl.: C12N 15/79, C12N 15/866, C07K 19/00, C07K 16/00

(54) **UTILISATION DU GENE CODANT LA CHAINE BETA DE LA PROTEINE C4BP DANS LA PRODUCTION DE PROTEINES DIMERIQUES RECOMBINANTES**
VERWENDUNG EINES FÜR DIE C4BP-PROTEIN-BETA-KETTE KODIERENDEN GENS ZUR HERSTELLUNG VON REKOMBINANTEN DIMEREN PROTEINEN
USE OF A GENE CODING FOR THE C4BP PROTEIN BETA CHAIN FOR PRODUCING RECOMBINANT DIMERIC PROTEINS

(30) Priorité: 22.04.2004 FR 0404295
(43) Date de publication de la demande: 03.01.2007
(73) Titulaire: Université De Reims Champagne-Ardenne, 51097 Reims Cedex (FR)
(72) Inventeur: COHEN, Jacques, Henri, Max, F-51100 Reims (FR); OUDIN, Stéphane, F-67200 Strasbourg (FR); DERVILLEZ, Xavier, 75015 Paris (FR); GIMENEZ, Annelise, F-51470 Saint Memmie (FR); DONVITO, Béatrice, F-51100 Reims (FR); TONYE-LIBYH, Marcelle, F-51100 Reims (FR)
(74) Mandataire: Desaix, Anne
(86) Numéro de dépôt international: PCT/FR2005/001007
(87) Numéro de publication internationale: WO 2005/106000

(56) Documents cités:
- FR-A- 2 736 916
- OUDIN S ET AL: "A soluble recombinant multimeric anti-Rh(D) single-chain Fv/CR1 molecule restores the immune complex binding ability of CR1-deficient erythrocytes" JOURNAL OF IMMUNOLOGY, THE WILLIAMS AND WILKINS CO. BALTIMORE, US, vol. 164, no. 3, 1 février 2000 (2000-02-01), pages 1505-1513, XP002231125 ISSN: 0022-1767
- LIBYH M T ET AL: "A recombinant human scFv anti-Rh(D) antibody with multiple valences using a C-terminal fragment of C4-binding protein" BLOOD, W.B.SAUNDERS COMPAGNY, ORLANDO, FL, US, vol. 90, no. 10, 15 novembre 1997 (1997-11-15), pages 3978-3983, XP002231124 ISSN: 0006-4971
- BLOM A M ET AL: "The C4b-binding protein-protein S interaction is hydrophobic in nature" BIOCHIMICA ET BIOPHYSICA ACTA. PROTEIN STRUCTURE AND MOLECULAR ENZYMOLOGY, ELSEVIER, AMSTERDAM,, NL, vol. 1388, no. 1, 14 octobre 1998 (1998-10-14), pages 181-189, XP004278525 ISSN: 0167-4838
- WEBB J.H., ET AL: "Role of CCP2 of the C4b-binding protein beta-chain in protein S binding evaluated by mutagenesis and monoclonal antibodies" EUR. J. BIOCHEM., vol. 270, 2003, pages 93-100, XP002289480

## Description

La présente demande décrit un procédé d'obtention de protéines dimériques recombinantes, utilisant un acide nucléique codant le fragment C-terminal de la chaîne β de la protéine C4BP comprenant les résidus d'acides aminés 193 à 252 ou un variant fonctionnel de ce fragment, ledit fragment permettant l'association covalente des polypeptides hétérologues auxquels il est fusionné. Sont également décrites les protéines dimériques susceptibles d'être obtenues par le procédé et les cellules pour la mise en oeuvre du procédé.

La possibilité de produire une protéine d'intérêt dans un système d'expression hétérologue constitue une application majeure de la biologie moléculaire. En particulier, la production de protéines d'intérêt dans des systèmes hétérologues permet d'obtenir des rendements de production élevés à moindre coût et de limiter les risques de contamination par des éléments indésirables, par exemple, virus ou prions comparativement aux méthodes de purification de protéines natives.

A titre d'exemples de biomolécules recombinantes d'intérêt en biotechnologie, on citera les antigènes, les anticorps ou leurs fragments, en particulier leurs fragments comportant tout ou partie des chaînes variables, les enzymes, les hormones, les cytokines ou les facteurs de croissance pour la production de vaccins, de kits de diagnostic ou de molécules à activité thérapeutique.

Parmi ces molécules biologiques, nombreuses sont celles qui, pour présenter l'activité biologique recherchée, voire pour présenter une activité biologique optimale, doivent s'assembler sous la forme d'un dimère. Pour leur production, il est donc nécessaire de s'assurer que l'étape de dimérisation est correctement effectuée. Selon la nature des monomères, l'assemblage en dimère a lieu spontanément après la synthèse des monomères, en général dans le réticulum endoplasmique, avant leur sécrétion dans le milieu extracellulaire. Cependant, dans certains cas, notamment lorsque les dimères sont mal ou non assemblés, il est nécessaire de recourir à une étape supplémentaire de couplage covalent des monomères. Des méthodes de couplages sont également requises pour la production d'hétérodimères particuliers.

On connaît dans l'état de la technique des méthodes de couplage utilisant un agent de pontage chimique pour l'obtention d'un conjugué. Le couplage est alors obtenu par des liaisons de type maléimide, succinimide, peptidique, disulfide et thioether. On peut en particulier se référer à l'ouvrage « Bioconjugate Techniques de Greg. T. HERMANSON (Academic Press, 1996) ».

Une méthode particulière consiste par exemple à ajouter, à une extrémité d'un peptide recombinant, un peptide hétérologue, dit peptide « linker », qui peut être aisément utilisé pour des liaisons disulfure, amine ou acide. Une autre approche consiste à coupler chimiquement un groupement biotinyl, qui permet de coupler ensuite toute substance liée à la streptavidine.

Pour une revue générale de ces méthodes de couplage, on pourra se référer par exemple à Methods of Immunological Analysis, Volume 2, René Masseyeff, Winfried Albert, Norman A. Staines VCH. Décembre 1992 *;* Handbook of Experimental immunology, Volume 1, 2nd Edition, Ed. D.M. Weir, Blackwell Scientific Publications, Oxford*.*

Les méthodes existantes, nécessitant une étape de réaction chimique supplémentaire à l'étape de synthèse proprement dite des monomères, sont toutefois mal adaptées à une production à grande échelle des protéines dimériques. En outre, pour certaines applications *in vivo,* il peut être nécessaire d'éviter l'utilisation d'agents pontants potentiellement immunogènes.

D'autres méthodes ont été proposées pour la production de protéines dimériques, consistant à produire un polypeptide de fusion, le polypeptide de fusion étant constitué du polypeptide d'intérêt et d'un domaine fonctionnel permettant la dimérisation.

Les domaines de dimérisation choisis sont par exemple les domaines des anticorps CL et CHI, la calmoduline ou le peptide liant la calmoduline correspondant, ou encore la streptavidine (Müller et al., FEBS Lett., 1998 422 : 259-264, Neri et al., BioTechnology, 1995, 13 : 373-377).

Enfin, la méthode la plus fréquemment employée consiste à exprimer un seul acide nucléique codant deux polypeptides reliés entre eux par un linker peptidique. Le linker peptidique présente cependant l'inconvénient d'être potentiellement immunogène et peut donc ne pas satisfaire à l'ensemble des applications visées pour une protéine dimérique, notamment une protéine destinée à être administrée à l'homme ou l'animal.

Il existe par conséquent un besoin d'identifier une méthode simple permettant la production de protéines recombinantes dimériques d'intérêt dont l'assemblage se fait spontanément sans recourir à une étape de polymérisation supplémentaire.

Il existe en outre un besoin d'identifier une méthode permettant la production de protéines recombinantes dimériques d'intérêt, dont l'association ne nécessite pas l'utilisation de molécules potentiellement immunogènes.

La présente description se propose de remédier, du moins en partie, aux inconvénients des méthodes décrites ci-dessus pour la production de protéines dimériques recombinantes.

La protéine C4BP est impliquée dans la coagulation et le système du complément. La forme majoritaire de la C4BP est composée de 7 chaînes α identiques de 75 Kd et d'une chaîne β de 45 Kd. Les chaînes α et β contiennent respectivement 8 et 3 domaines SCR (Short Consensus Repeat), ces motifs étant retrouvés dans de nombreuses protéines régulatrices du complément et constitués de 50-70 acides aminés organisés en feuillets β.

Le rôle de la chaîne α dans la polymérisation de la protéine C4BP a été étudié par Kask et al. (Biochemistry, 2002, 41, 9349-9357). Ces auteurs ont en particulier montré que la partie C-terminale de la chaîne α, notamment sa structure en hélice α et la présence de deux cystéines, est nécessaire à la polymérisation de la protéine C4BP, lorsque la chaîne α est exprimée dans un système hétérologue.

On a par ailleurs décrit la production de polymères constitués de sous-unités de fragment d'anticorps ou de récepteur du complément CR1 fusionnés à la partie C-terminale de la chaîne α de C4BP (Libyh et al., Blood, 1997, 90 : 3978-3983 ; Oudin et al., J. Immunol., 2000, 164, 1505-1513).

Les demandes CA 2 227 030 et FR 2 736 916 A décrivent également la production de protéines hétéromultimériques recombinantes par l'utilisation des fragments C-terminaux des chaînes α et β de la protéine C4BP en fusion avec des polypeptides d'intérêt.

A la connaissance de la Demanderesse, l'utilisation de la chaîne β de la protéine C4BP, indépendamment de son utilisation en association avec la chaîne α de la protéine C4BP, n'a en revanche jamais été décrite pour la production de protéines multimériques, et en particulier pour la production de protéines dimériques.

Les inventeurs ont maintenant constaté de manière surprenante, que l'expression dans un système hétérologue, d'un acide nucléique codant en fusion le fragment C-terminal de la chaîne β de la protéine C4BP et un polypeptide d'intérêt, permettait la production de protéines dimériques recombinantes, dont les monomères sont liés de manière covalente.

Ainsi, la demande décrit un procédé d'obtention d'une protéine dimérique recombinante, comprenant :
a) la transfection de cellules hôtes par un vecteur permettant l'expression d'une séquence nucléotidique codant un polypeptide de fusion, ledit polypeptide de fusion comprenant au moins le fragment constitué des acides aminés des positions 193 à 252 de la chaîne β de la protéine C4BP humaine ou un variant fonctionnel de ce fragment par délétion, addition ou substitution d'un ou plusieurs acides aminés, conservant la capacité de former une protéine au moins dimérique, ledit polypeptide de fusion comprenant en outre un polypeptide hétérologue à ladite chaîne β,
b) la culture des cellules transfectées dans des conditions appropriées pour l'expression de la séquence nucléotidique codant le polypeptide de fusion et l'association covalente de deux polypeptides de fusion *in vivo* pour former une protéine dimérique,
c) la récupération des protéines dimériques formées.

Afin d'obtenir une protéine dimérique et non hexa- ou heptamérique, de préférence le système hétérologue ne doit pas contenir d'acide nucléique permettant l'expression ou une surexpression du fragment C-terminal de la chaîne α de la protéine C4BP, impliquée dans la polymérisation de la C4BP.

Par « polypeptide hétérologue à la chaîne β », il faut comprendre tout polypeptide caractérisé par le fait que la séquence de ce polypeptide n'est pas naturellement associée au fragment de la chaîne β auquel il est fusionné. De préférence, il s'agit d'un polypeptide qui ne présente pas seul, la capacité de s'associer de façon covalente avec un autre polypeptide pour former un homodimère ou un hétérodimère.

On citera à titre d'exemples de polypeptide hétérologue, des enzymes, des facteurs de régulation d'activité enzymatique, des ligands de récepteurs, des haptènes, des antigènes, des anticorps, des fragments d'anticorps, des récepteurs, notamment des récepteurs monochaînes ou des accepteurs protéiques de médicament.

Bien entendu, l'homme du métier choisira la séquence d'un polypeptide hétérologue en fonction de l'application qu'il souhaite. Les séquences des polypeptides peuvent être choisies par exemple parmi les principes actifs de médicaments, incluant les immunotoxines, les antioxydants, les antibiotiques, les facteurs de croissance, les hormones intracellulaires, les cytokines, les toxines, les neuromédiateurs, les agents antimicrobiens, en particulier, antiviraux, antibactériens et antiparasitaires, ou antitumoraux ou encore tout autre agent thérapeutique ou prophylactique d'intérêt.

Les séquences de polypeptides seront choisies notamment parmi les immunoglobulines et les fragments d'anticorps, en particulier les fragments correspondant aux domaines variables ou à des parties actives immunologiquement de ces domaines, telles que les scFv (single chain Fragment variable), les enzymes dimériques comme la fumarylacétoacétate hydrolase, ou encore les récepteurs dimériques comme le récepteur pour le C3Bi (CD11+CD18).

La séquence polypeptidique de la chaîne β de la protéine C4BP humaine est décrite dans Hillarp et Dahlbäck (1990, PNAS, Vol. 87, pp 1183-1187), ainsi que la séquence de l'ADNc la codant.

Une séquence de la chaîne β de la protéine C4BP et son ADNc est également décrite dans la base de données NCBI sous le numéro d'accession NM_000716 dont la numérotation protéique est utilisée ici.

Le fragment correspondant aux positions 193-252 est identifié par référence à cette séquence et peut par exemple être obtenu par amplification PCR à partir d'une banque d'ADNc humain en choisissant des amorces hybridant spécifiquement aux extrémités correspondantes de la séquence codant le fragment souhaité de la chaîne β. Il peut alternativement être obtenu par toute méthode appropriée connue de l'homme du métier.

Pour simplifier la lecture, dans le texte qui suit, on entend par le terme « fragment 193-252 » ou « fragment 193-252 de la chaîne β », sauf précision spécifique, le fragment constitué de l'enchaînement consécutif des acides aminés des positions 193 à 252 de la chaîne β de la protéine C4BP humaine.

La présente invention a ainsi trait à un procédé d'obtention d'une protéine dimérique recombinante, comprenant :
a. la transfection de cellules hôtes par un vecteur permettant l'expression d'une séquence nucléotidique codant un polypeptide de fusion, ledit polypeptide de fusion comprenant au moins le fragment constitué des acides aminés des positions 193 à 252 de la chaîne β de la protéine C4BP humaine ou un variant fonctionnel de ce fragment, ledit variant fonctionnel conservant la capacité de former une protéine au moins dimérique et étant choisi dans le groupe constitué par :
   a1) une séquence modifiée du fragment 193-252 de la chaîne β, dont moins de 25% des acides aminés du fragment 193-252, de préférence moins de 10%, ont été supprimés ou remplacés, dans laquelle les cystéines situées aux positions 202 et 216, ainsi qu'au moins 3 acides aminés en amont et en aval de chaque cystéine, ont été conservés ;
   a2) une séquence modifiée du fragment 193-252 de la chaîne β, dans laquelle une cystéine responsable de la dimérisation est substitué en un acide aminé choisi parmi l'alanine, la valine, la phénylalanine, la proline, la méthionine, l'isoleucine, la leucine et le tryptophane, et un autre acide aminé du fragment est substitué par une cystéine ;
   a3) une séquence modifiée du fragment 193-252 de la chaîne β par insertion d'une séquence hétérologue à la chaîne β, entre les cystéines responsables de la dimérisation ; et
   a4) une séquence modifiée du fragment 193-252 de la chaîne β, par suppression d'acides aminés entre les cystéines responsables de la dimérisation ;
   ledit polypeptide de fusion comprenant en outre un polypeptide hétérologue à ladite chaîne β,
b. la culture des cellules transfectées dans des conditions appropriées pour l'expression de la séquence nucléotidique codant le polypeptide de fusion et l'association covalente de deux polypeptides de fusion *in vivo* pour former une protéine dimérique, et
c. la récupération des protéines dimériques formées.

Dans un mode de réalisation préféré, le fragment 193-252 de la chaîne β de la protéine C4BP humaine a la séquence polypeptidique suivante :

Une séquence d'acide nucléique correspondant à cette séquence polypeptidique est également décrite par Hillarp et Dahlbäck (1990, PNAS, Vol. 87, pp 1183-1187).

Pour la production de protéines dimériques recombinantes ici décrite, l'homme du métier peut utiliser également une séquence codant un variant fonctionnel du fragment 193-252, conservant la capacité de former au moins un dimère, par exemple un homodimère ou hétérodimère, un trimère, un tétramère ou tout multimère contenant un nombre varié de polypeptides de fusion.

Au sens de l'invention, on entend par l'expression « variant fonctionnel du fragment 193-252 », une séquence polypeptidique modifiée par rapport à la séquence du fragment 193-252 de la chaîne β, par délétion, substitution ou addition d'un ou plusieurs acides aminés, ladite séquence modifiée conservant néanmoins la capacité de former des protéines au moins dimériques par le procédé de l'invention. Plus précisément, la production de protéines dimériques en utilisant une séquence codant un variant fonctionnel du fragment 193-252 doit être d'au moins 80% égale à celle obtenue avec une séquence native codant le fragment 193-252, de préférence au moins 90%, dans un système d'expression identique. De préférence, le variant est tel que plus de 80% des polypeptides de fusion qui le contiennent sont produits sous forme de dimères dans un système d'expression eucaryote selon l'invention.

Dans un premier mode de réalisation particulier, un variant fonctionnel correspond en particulier à un fragment de la chaîne β contenant le fragment 193-252 et contenant également une séquence adjacente de la chaîne β en amont de ce fragment, incluant par exemple la dernière séquence SCR (résidus 136-192) et/ou 4 ou 5 acides aminés [GS]. Des séquences codant des fragments plus longs de la chaîne β, voire toute la chaîne β, peuvent également être utilisées. Pour certaines applications, il est préférable d'éviter d'utiliser une séquence codant une chaîne β capable de lier la protéine S participant à la coagulation. Si la séquence choisie code pour un fragment contenant les deux premiers motifs SCR de la chaîne β, ceux-ci seront de préférence des versions mutées par addition, délétion ou substitution d'acides aminés, de sorte à supprimer la possibilité d'interaction avec la protéine S. L'addition des motifs SCR et/ou domaines [GS] peut être réalisée dans le but de modifier, par exemple d'augmenter, la flexibilité du polypeptide de fusion ainsi obtenu ou encore pour permettre au polypeptide de fusion ou au polypeptide hétérologue d'adopter une conformation appropriée pour la formation des multimères, particulièrement des dimères, et appropriée à son activité biologique (fixation, catalyse de la réaction enzymatique, interaction avec le médicament).

Dans un mode de réalisation particulier, une séquence codant un variant du fragment 193-252 de la chaîne β est une séquence dont l'acide nucléique correspondant est capable d'hydrider dans des conditions stringentes avec la séquence codant le fragment 193-252 telle que décrite par Hillarp et Dahlbäck (1990, PNAS, Vol. 87, pp 1183-1187).

Par « conditions stringentes », on entend les conditions qui permettent l'hybridation spécifique de deux séquences d'ADN simple brin à environ 65°C par exemple dans une solution de 6 x SSC, 0,5% SDS, 5X Denhardt's solution et 100 µg d'ADN non spécifique ou toute autre solution de force ionique équivalente et après un lavage à 65°C, par exemple dans une solution d'au plus 0,2 x SSC et 0,1% SDS ou toute autre solution de force ionique équivalente.

De préférence, la séquence de nucléotides (polynucléotide) codant pour un variant fonctionnel du fragment 193-252 susdit et hybridant dans des conditions stringentes avec la séquence codant ledit fragment a, dans la partie qui hybride, une longueur au moins égale à 50%, de préférence au moins 80%, de la longueur de la séquence codant le fragment 193-252. Dans un mode de réalisation particulier, la séquence de nucléotides (polynucléotide) codant pour un variant fonctionnel du fragment 193-252 susdit et hybridant dans des conditions stringentes avec la séquence codant ledit fragment a, dans la partie qui hybride, substantiellement la même longueur que la séquence codant le fragment 193-252 susdit.

Dans un autre mode de réalisation, un variant fonctionnel est une séquence modifiée du fragment 193-252, dont un ou plusieurs acides aminés non essentiels à la fonction de dimérisation ont été supprimés ou substitués et/ou un ou plusieurs acides aminés essentiels à la dimérisation ont été remplacés par des acides aminés de groupes fonctionnels équivalents (substitution conservative). Il est en particulier recommandé que les deux cystéines, situées aux positions 202 et 216, ainsi que la structure peptidique autour de ces cystéines, soient conservées pour permettre la formation de ponts disulfures nécessaires à la dimérisation, par exemple par la conservation d'au moins 3 acides aminés en amont et en aval de chaque cystéine.En particulier, un variant fonctionnel peut aussi être obtenu en insérant une séquence hétérologue à la chaîne β, et notamment des domaines de la chaîne α de C4BP, entre les cystéines responsables de la dimérisation, ou au contraire en supprimant certains acides aminés présents entre ces mêmes cystéines. Alternativement, on peut réaliser un variant fonctionnel par la modification ponctuelle de certains acides aminés, et en particulier la substitution d'une cystéine responsable de la dimérisation par un acide aminé neutre en terme d'implication dans le processus de dimérisation (par exemple les acides aminés A, V, F, P, M, I, L et W), et dans le même temps la substitution d'un autre acide aminé par une cystéine de façon à conserver la capacité de formation de ponts disulfures intracaténaires et/ou intercaténaires entre les cystéines. Ces modifications conduisent ainsi à la variation de l'écartement entre les différentes cystéines impliquées dans le processus de multimérisation, notamment dimérisation.

Les modifications apportées à la chaîne β de la protéine C4BP peuvent notamment être déterminées pour modifier, en particulier améliorer, l'accessibilité du polypeptide hétérologue dans le polypeptide de fusion, par exemple en modifiant la flexibilité du polypeptide de fusion.

De façon préférée, moins de 50% des acides aminés du fragment 193-252 sont ainsi supprimés ou remplacés, et mieux, moins de 25%, voire encore mieux, moins de 10% (par exemple 5 acides aminés ou moins) ou moins de 5% (1 ou 2 acides aminés).

Les séquences codant le fragment C-terminal de la chaîne β et codant le polypeptide d'intérêt sont fusionnées et clonées dans un vecteur d'expression approprié. De préférence, le polypeptide de fusion codé par la séquence nucléotidique contient dans sa partie N-terminale, le polypeptide hétérologue et dans sa partie C-terminale, le fragment de la chaîne β de la protéine C4BP.

Le vecteur d'expression est choisi en fonction de la cellule hôte dans laquelle la construction est introduite. De préférence, le vecteur d'expression est choisi parmi les vecteurs permettant l'expression dans des cellules eucaryotes, et notamment parmi des vecteurs chromosomiques, ou épisomiques ou dérivés de virus, et en particulier des vecteurs dérivés de plasmides, de chromosomes de levures, ou de virus tels que des baculovirus, des papovirus ou SV40, des rétrovirus, ou leurs combinaisons, notamment des phagemides et des cosmides. De préférence, il s'agit d'un vecteur permettant l'expression de baculovirus, capable d'infecter des cellules d'insectes.

Le cas échéant, la séquence codant le polypeptide de fusion comprend également, préférentiellement dans sa partie 5', une séquence codant un peptide signal pour la sécrétion du polypeptide de fusion. Classiquement, la séquence d'un peptide signal est une séquence de 15 à 20 acides aminés, riche en acides aminés hydrophobes (Phe, Leu, Ile, Met et Val). A titre d'exemples de peptide signal, on citera en particulier la séquence signal gp67 telle qu'utilisée dans le vecteur d'expression choisi dans la partie expérimentale.

Le vecteur comprend toutes les séquences nécessaires à l'expression de la séquence codant le polypeptide de fusion. En particulier, il comprend un promoteur approprié, choisi en fonction de la cellule dans laquelle la construction doit être introduite.

Un exemple de vecteur d'expression pour baculovirus est décrit dans la partie expérimentale.

Au sens de l'invention, on entend par « cellule hôte », une cellule capable d'exprimer un gène porté par un acide nucléique hétérologue à la cellule et qui a été introduit dans le génome de la cellule par une méthode de transfection. De préférence, une cellule hôte est une cellule eucaryote. Une cellule hôte eucaryote est en particulier sélectionnée parmi les cellules de levures, telles que *S. cerevisiae,* les cellules de champignons filamenteux, telles que *Aspergillus sp.,* les cellules d'insectes telles que les cellules S2 de *Drosophila,* ou Sf9 de *Spodoptera,* les cellules de mammifères ou les cellules de plantes.

Parmi les cellules de mammifères, on citera en particulier, les lignées cellulaires de mammifères, telles que les cellules CHO, COS, HeLa, C127, 3T3, HepG2 ou encore les cellules L(TK-).

Dans un mode de mise en oeuvre préféré, lesdites cellules hôtes sont choisies parmi des lignées cellulaires eucaryotes, de préférence il s'agit des cellules d'insectes Sf9.

Toute méthode de transfection connue de l'homme du métier pour l'obtention de cellules exprimant un acide nucléique hétérologue peut être utilisée pour la mise en oeuvre de l'étape (a) du procédé. Des méthodes de transfection sont par exemple décrites dans Sambrook et al., 2001 (Molecular Cloning : A laboratory Manual, 3rd Ed., Cold Spring Harbor, laboratory press, Cold Spring Harbor, New York).

Dans un mode de réalisation particulier du procédé de l'invention, la cellule hôte permet la co-expression de deux polypeptides de fusion, un premier polypeptide de fusion A, constitué de la fusion d'un fragment 193-252 ou d'un variant fonctionnel de ce fragment avec un polypeptide d'intérêt A', et un second polypeptide de fusion B, constitué de la fusion d'un fragment 193-252 ou d'un variant fonctionnel de ce fragment avec un polypeptide d'intérêt B'. Dans ce mode de réalisation particulier, la co-expression des deux polypeptides de fusion permet outre la production des homodimères A-A et B-B, la production d'hétérodimères A-B.

Les polypeptides d'intérêt A' et B' sont choisis, indépendamment l'un de l'autre, dans le groupe constitué des enzymes, des facteurs de régulation d'activité enzymatique, des ligands de récepteurs, des haptènes, des antigènes, des anticorps, des fragments d'anticorps, des médicaments, des récepteurs, notamment des récepteurs monochaînes ou des accepteurs protéiques de médicament.

L'expression « choisis indépendamment l'un de l'autre » signifie que les deux polypeptides d'intérêt A' et B' (ou polypeptides hétérologues) peuvent être de nature différente, c'est à dire par exemple l'un est une enzyme, l'autre un récepteur ou au contraire de même nature, c'est à dire deux ligands, deux anticorps ou de récepteurs monochaînes. Lorsque la nature des polypeptides hétérologues est identique, il n'en demeure pas moins que les deux polypeptides peuvent être différents par exemple dans leur composition protéique ou dans leurs modifications post-traductionnelles ; Ainsi, le procédé d'obtention permet également la production d'hétérodimères, dans lesquels les premier et second polypeptides hétérologues de ces hétérodimères comportent chacun un site anticorps différent de celui de l'autre polypeptide, un ligand différent de celui de l'autre polypeptide ou un récepteur monochaîne différent de celui de l'autre polypeptide.

Par le terme « différent » quand on se réfère à des exemples de polypeptides hétérologues, on entend une séquence protéique dont la séquence primaire (séquence en acides aminés) de l'un des polypeptides hétérologues est différente par au moins un acide aminé de la séquence primaire de l'autre polypeptide. Par exemple, les séquences primaires peuvent être différentes pour l'ensemble des acides aminés bien que les protéines soient de même nature, par exemple deux enzymes, deux antigènes ou deux haptènes. Alternativement, le terme « différent » couvre également des polypeptides hétérologues ayant la même séquence primaire, mais possédant des modifications post-traductionnelles différentes, par exemple en terme d'acétylation, d'amidation, de biotinylation, de carboxylation, d'hydroxylation, de méthylation, de phosphorylation ou de sulfatation, ou par l'ajout de lipides (isoprénylation, palmitoylation et myristoylation), de glucides (glycosylation) ou de polypeptides (ubiquitination).

Par l'expression « site anticorps », on entend les séquences protéiques nécessaires à la reconnaissance d'un antigène donné, et notamment les domaines variables (des chaînes lourdes et/ou légères) d'une immunoglobuline.

Par « accepteur protéique de médicament », on entend tout acide aminé, chaîne peptidique (au moins deux acides aminés consécutifs) ou ensemble d'acides aminés (au moins deux acides aminés non consécutifs) interagissant avec un médicament.

Ainsi, l'invention porte également sur un procédé d'obtention d'hétérodimères, ledit procédé comprenant :
a. la transfection de cellules hôtes par un ou plusieurs vecteurs permettant l'expression d'une ou plusieurs séquences nucléotidiques codant :
   i. un premier polypeptide de fusion, ledit polypeptide de fusion comprenant le fragment 193-252 de la chaîne β de la protéine C4BP humaine ou un variant fonctionnel de ce fragment et un premier polypeptide hétérologue à ladite chaîne β, et
   ii. un second polypeptide de fusion, ledit polypeptide de fusion comprenant au moins le fragment 193-252 de la chaîne β de la protéine C4BP humaine ou un variant fonctionnel de ce fragment et un second polypeptide hétérologue à ladite chaîne β dont la séquence est différente de celle du premier polypeptide hétérologue,
b. la culture des cellules transfectées dans des conditions appropriées pour l'expression de la ou des séquences nucléotidiques codant le premier et le second polypeptides de fusion et l'association de deux polypeptides de fusion *in vivo* pour former une protéine hétérodimérique,
c. la récupération des protéines hétérodimériques formées.

L'invention vise également une protéine dimérique ou hétérodimérique recombinante, susceptible d'être obtenue par l'un des procédés d'obtention décrits ci-dessus.

L'invention porte en particulier sur une protéine dimérique recombinante caractérisée en ce qu'elle est constituée de deux polypeptides de fusion, chaque polypeptide de fusion comprenant au moins le fragment 193-252 de la chaîne β de la protéine C4BP humaine ou un variant fonctionnel de ce fragment et un polypeptide hétérologue à ladite chaîne β.

Dans un mode de réalisation particulier, la protéine dimérique recombinante selon l'invention est caractérisée en ce que lesdits polypeptides de fusion sont associés par liaison covalente entre deux cystéines du fragment de la chaîne β de la protéine C4BP.

De préférence, chaque monomère de la protéine dimérique comprend le fragment de la chaîne β de la protéine C4BP fusionné à l'extrémité C-terminale d'un polypeptide hétérologue à ladite chaîne β.

Dans un mode de réalisation particulier, le polypeptide hétérologue est choisi dans le groupe constitué des enzymes, des facteurs de régulation d'activité enzymatique, des ligands de récepteurs, des haptènes, des antigènes, des anticorps, des fragments d'anticorps, des médicaments, des récepteurs, notamment des récepteurs monochaînes ou des accepteurs protéiques de médicament. Par exemple, le fragment d'anticorps comporte tout ou partie des régions variables d'un anticorps, fonctionnel pour la liaison Antigène-Anticorps.

L'invention porte également sur une protéine hétérodimérique recombinante, caractérisée en ce qu'elle est constituée d'un premier et d'un second polypeptides de fusion, chaque polypeptide de fusion comprenant au moins un fragment constitué des acides aminés des positions 193 à 252 de la chaîne β de la protéine C4BP humaine ou un variant fonctionnel de ce fragment par délétion, addition ou substitution d'un ou plusieurs acides aminés conservant la capacité de former une protéine au moins dimérique, ledit premier polypeptide de fusion comprenant en outre un polypeptide hétérologue à ladite chaîne β, et le second polypeptide de fusion comprenant un second polypeptide hétérologue à ladite chaîne β différent du premier polypeptide hétérologue.

Dans un mode de réalisation particulier, le fragment de la chaîne β de la protéine C4BP humaine, comprenant au moins les acides aminés des positions 193 à 252, du premier et du second polypeptides de fusion, ou un variant fonctionnel de ce fragment, sont respectivement fusionnés à l'extrémité C-terminale du premier polypeptide et du second polypeptides hétérologues.

Les premier et second polypeptides hétérologues sont choisis, indépendamment l'un de l'autre, dans le groupe constitué des enzymes, des facteurs de régulation d'activité enzymatique, des ligands de récepteurs, des haptènes, des antigènes, des anticorps, des fragments d'anticorps, des médicaments, des récepteurs, notamment des récepteurs monochaînes ou des accepteurs protéiques de médicament.

Ainsi, les hétérodimères sont caractérisés en ce le premier et le second polypeptides hétérologues sont des ligands différents, des sites anticorps différents ou des récepteurs monochaînes différents.

Dans un mode de réalisation préféré, la protéine hétérodimérique comprend un premier polypeptide hétérologue qui est un anticorps et un second polypeptide hétérologue qui est un médicament. Dans un autre mode de réalisation préféré, la protéine hétérodimérique comprend un premier polypeptide hétérologue qui est un anticorps et un second polypeptide hétérologue qui est un accepteur protéique de médicament.

Dans un mode de réalisation particulier, la protéine hétérodimérique recombinante selon l'invention est caractérisée en ce que lesdits polypeptides de fusion sont associés par liaison covalente entre deux cystéines du fragment de la chaîne β de la protéine C4BP.

L'invention concerne également une cellule eucaryote recombinante permettant la synthèse d'une protéine dimérique ou hétérodimérique telle que définie ci-dessus, et caractérisée en ce qu'elle est susceptible d'être obtenue par la mise en oeuvre de l'étape (a) du procédé d'obtention de l'invention définie plus haut. Dans un mode de réalisation particulier, la cellule eucaryote recombinante selon l'invention est une cellule d'insecte, de préférence une lignée cellulaire Sf9.

L'invention porte également sur l'utilisation, dans un procédé de production d'une protéine dimérique recombinante, d'un acide nucléique codant un polypeptide de fusion, ledit polypeptide de fusion comprenant au moins le fragment 193-252 de la chaîne β de la protéine C4BP ou un variant fonctionnel de ce fragment et un polypeptide hétérologue à ladite chaîne β.

Enfin, l'invention concerne l'utilisation, dans un procédé de production d'une protéine hétérodimérique recombinante, de deux acides nucléiques :
a) un premier acide nucléique codant un premier polypeptide de fusion, ledit premier polypeptide de fusion comprenant au moins le fragment constitué des acides aminés des positions 193 à 252 de la chaîne β de la protéine C4BP humaine ou un variant fonctionnel de fragment par addition, délétion ou substitution d'un ou plusieurs acides aminés, conservant la capacité de former une protéine hétérodimérique, ledit premier polypeptide de fusion comprenant en outre un premier polypeptide hétérologue à ladite chaîne β, et
b) un second acide nucléique codant un second polypeptide de fusion, ledit second polypeptide de fusion comprenant au moins le fragment constitué des acides aminés des positions 193 à 252 de la chaîne β de la protéine C4BP humaine ou un variant fonctionnel de fragment par addition, délétion ou substitution d'un ou plusieurs acides aminés, conservant la capacité de former une protéine hétérodimérique, ledit second polypeptide de fusion comprenant en outre un second polypeptide hétérologue à ladite chaîne β dont la séquence est différente de celle du premier polypeptide hétérologue.

La protéine C4BP utilisée pour la réalisation de l'invention est avantageusement la protéine C4BP humaine.

Les exemples qui suivent permettent d'illustrer certains modes de réalisation préférés du procédé de l'invention et de comprendre plus aisément sa mise en oeuvre.

### DESCRIPTION DES FIGURES

La figure 1 est un schéma du plasmide PAcgp76c dans lequel a été introduit l'acide nucléique codant le polypeptide de fusion constitué du site de reconnaissance de la GPA (glycophorine A) (scFv) et d'un fragment C-terminal de la chaîne β de la protéine C4BP (C4BPBeta).
La figure 2 est un autoradiogramme d'un Western Blot après révélation à l'aide d'un anticorps anti-scFv marqué.
   Puits 1 : surnageant de culture de cellules sF9 infectées par un virus contenant l'acide nucléique codant scFv anti-GPA fusionné avec le fragment C-terminal de la chaîne β de la protéine C4BP. Production de monomères et diméres (en majorité) scFv/C4BPβ.
   Puits 2 : surnageant de culture de cellules sF9 infectées par un virus contenant l'acide nucléique codant ScFv anti-GPA en fusion avec le fragment C-terminal de la chaîne α de la protéine C4BP. Production d'heptamères scFv/C4BPα.
   Puits 3 : surnageant de culture de cellules sF9 infectées par un virus contenant l'acide nucléique codant une protéine recombinante non relatée, Témoin négatif.

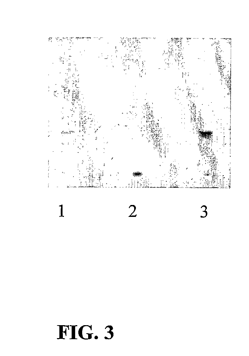

La figure 3 illustre le résultat du test d'agglutination
   Colonne 1 : Anticorps anti-glycophorine A, Témoin positif
   Colonne 2 : Surnageant cellules sF9 infectées par un virus recombinant (protéine non relatée), Témoin négatif
   Colonne 3 : Surnageant cellules sF9 infectées par un virus recombinant scFv antiGPA/C4BPβ, agglutination des hématies.

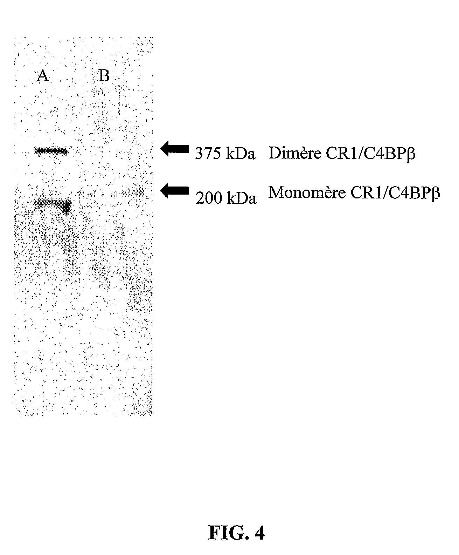

La figure 4 est un autoradiogramme d'un gel de protéines de surnageants après immunoprécipitation à l'aide d'un anticorps anti-CR1 J3D3. La piste A montre une électrophorèse en conditions non réductrices et la piste B montre une électrophorèse en conditions réductrices.

### EXEMPLES : Production d'une protéine recombinante dimérique anti-GPA dans des cellules d'insectes Sf9

### 1. Préparation du plasmide permettant l'expression d'un polypeptide de fusion

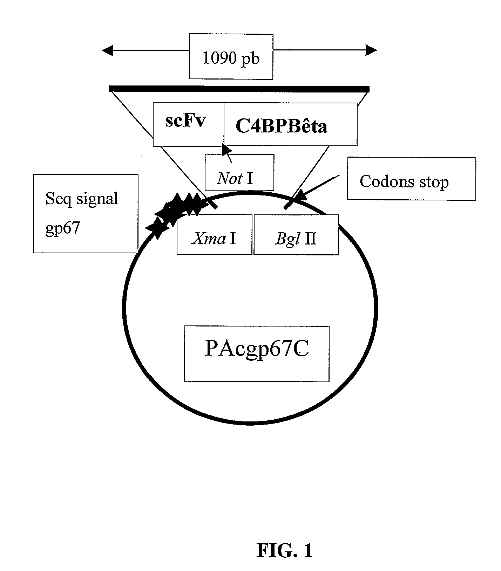

Une séquence codant un polypeptide de fusion constitué du site de reconnaissance de l'anticorps anti-GPA (R18) (ScFv) et d'un fragment C-terminal de la chaîne β (résidus 137 à 252 de la chaîne β) a été introduite dans le vecteur d'expression pour baculovirus (pAcgp67) (Pharmingen, San Diego, USA ; Stewart L.M.D, Hirst M., Nature 1991 ; 352 :85-88). La figure 1 présente de façon schématique le plasmide obtenu.

### 2. Transfection des cellules d'insectes Sf9

La plasmide a été co-transfecté avec l'ADN Baculogold linéarisé (Becton Dickinson, Le Pnt de Claix, France) dans les cellules d'insectes Sf9 en vue d'obtenir des virus recombinants.

### 3. Production de protéines dimériques ScFv anti-GPA/C4BPβ dans des cellules d'insectes Sf9

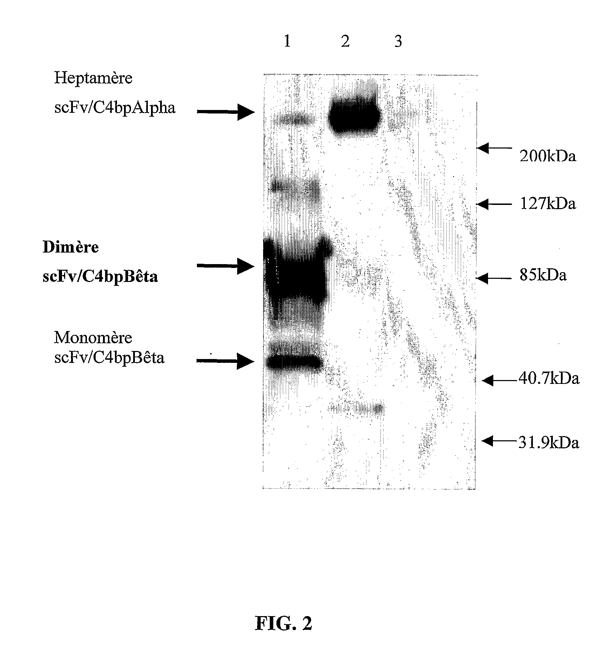

Les cellules d'insectes (6.10⁶ cellules Sf9) ont été infectées par les virus recombinants (6.10⁷ virus) dans 10 ml de milieu de culture et incubées 5 jours à 27°C selon la méthode classique par exemple décrite dans le manuel d'instruction « Baculovirus Expression System », May 99, Pharmingen, San Diego, USA, et dans Guide to « Baculovirus Expression Vector Systems and Insect Cell Culture Techniques », Invitrogen, Cergy Pontoise France. Les surnageants de culture ont ensuite été concentrés 15 fois sur centricon YM-20 (Millipore, St Quentin en Yvelines, France) puis soumis à une électrophorèse SDS-PAGE sur gel à gradient 4-15% Tris-HCl (BioRad, Marnes la Coquette, France) en conditions natives. Le Western Blot a été révélé par un anticorps anti-ScFv. Les résultats sont présentés à la figure 2. Le résultat montre que la majorité des polypeptides contenant le fragment ScFv en fusion avec le fragment C-terminal de la chaîne β (puits 1) migrent sous la forme de protéines dimériques d'un poids moléculaire d'environ 85 kDa. Les polypeptides contenant le fragment ScFv en fusion avec le fragment C-terminal de la chaîne α (puits 2) migrent sous la forme de protéines hetpamériques d'un poids moléculaire supérieur à 200 kDa.

4. Estimation de la concentration en protéines dimériques scFv/C4BPβ Les surnageants ont été récupérés 4 jours (J4) et 6 jours (J6) après l'infection. Le dosage du milieu et des surnageants préalablement concentrés a été réalisé au spectrophomètre à 280nm.

Les protéines sont soumises à une électrophorèse sur gel de polyacrylamide dénaturant au SDS (acrylamide 4-15%, Tris HCl) puis ce gel est coloré au bleu de Coomassie. Une mesure est ensuite réalisée sur le gel afin d'estimer le pourcentage de la quantité des protéines contenues dans chaque bande par rapport à la quantité totale de protéines. La concentration en protéines totales obtenues est la suivante :
A J4, 0,65 g/l de protéines ScFv β.
A J6, 0,63 g/l de protéines ScFv β.

Le pourcentage du pic correspondant à la bande d'intérêt est évalué à 5,7% de la quantité totale à J4, et 7,3% de la quantité totale à J6, soit 47 mg/litre de protéines dimériques d'intérêt produites.

Ainsi, rapporté au nombre de cellules, à J6, le rendement obtenu est d'environ 76µg de protéines dimériques /10⁶ cellules.

Le modèle ScFv antiglycophorine A montre qu'il est possible de produire des protéines dimériques recombinantes dans des cellules d'insecte, et d'obtenir leur sécrétion dans une seule forme moléculaire, à une concentration d'intérêt pratique.

### 5. Test fonctionnel de la protéine scFv/C4BPβ : hémagglutination

Afin de vérifier que les dimères produits sont fonctionnels, il a été vérifié que les protéines produites étaient capables d'agglutiner les érythrocytes et donc de reconnaître la glycophorine A.

Pour ce faire, les globules rouges sont lavés trois fois dans du PBS. Les surnageants de culture sont mis en contact avec les hématies, incubés 45 minutes à 37°C puis mis sur colonne DiaMed-ID NaCl, Enzyme test and cold agglutinins (DiaMed, Paris) et centrifugés.

Le résultat présenté à la figure 3 montre que les protéines produites sont capables d'agglutiner les érythrocytes et sont donc fonctionnelles (colonne 3).

### 6. Production d'une protéine dimérique CR1/C4BPβ

Le CR1 (complément Receptor 1) est une glycoprotéine transmembranaire des érythrocytes qui intervient dans la capture et l'élimination des complexes immuns. La densité de CR1 est diminuée dans des pathologies comme le SIDA ou le Lupus Erythémateux Disséminé.

Les cellules CHO transfectées avec un vecteur d'expression pKC3 (J. Virol., 50, 1984, 606-614) exprimant un polypeptide de fusion CR1/C4BPβ ont été cultivées une nuit en présence de ³²Sméthionine-cystéine. Les surnageants de culture sont ensuite immuno-précipités par l'anticorps monoclonal anti-CR1 J3D3. Les protéines immuno-précipitées sont ensuite soumises à une électrophorèse PAGE-SDS (4% d'acrylamide) en conditions non réductrices (piste A) et réductrices (piste B) (voir figure 4). Deux protéines différentes sont immunoprécipitées, une d'un poids moléculaire de 200 kDa (monomère) et l'autre d'un poids moléculaire de 375 kDa (dimère). Une fois réduite, ces deux molécules ont le même poids moléculaire.

Ces résultats confirment la production de protéines majoritairement sous la forme de dimères par le procédé ici décrit.

## Revendications

1. Procédé d'obtention d'une protéine dimérique recombinante, comprenant :
a. la transfection de cellules hôtes par un vecteur permettant l'expression d'une séquence nucléotidique codant un polypeptide de fusion, ledit polypeptide de fusion comprenant au moins le fragment constitué des acides aminés des positions 193 à 252 de la chaîne β de la protéine C4BP humaine ou un variant fonctionnel de ce fragment, ledit variant fonctionnel conservant la capacité de former une protéine au moins dimérique et étant choisi dans le groupe constitué par :
a1) une séquence modifiée du fragment 193-252 de la chaîne β, dont moins de 25% des acides aminés du fragment 193-252, de préférence moins de 10%, ont été supprimés ou remplacés, dans laquelle les cystéines situées aux positions 202 et 216, ainsi qu'au moins 3 acides aminés en amont et en aval de chaque cystéine, ont été conservés ;
a2) une séquence modifiée du fragment 193-252 de la chaîne β, dans laquelle une cystéine responsable de la dimérisation est substitué en un acide aminé choisi parmi l'alanine, la valine, la phénylalanine, la proline, la méthionine, l'isoleucine, la leucine et le tryptophane, et un autre acide aminé du fragment est substitué par une cystéine ;
a3) une séquence modifiée du fragment 193-252 de la chaîne β par insertion d'une séquence hétérologue à la chaîne β, entre les cystéines responsables de la dimérisation ; et
a4) une séquence modifiée du fragment 193-252 de la chaîne β, par suppression d'acides aminés entre les cystéines responsables de la dimérisation ;
ledit polypeptide de fusion comprenant en outre un polypeptide hétérologue à ladite chaîne β,
b. la culture des cellules transfectées dans des conditions appropriées pour l'expression de la séquence nucléotidique codant le polypeptide de fusion et l'association covalente de deux polypeptides de fusion *in vivo* pour former une protéine dimérique, et
c. la récupération des protéines dimériques formées.

2. Procédé selon la revendication 1, **caractérisé en ce que** les cellules transfectées ne contiennent pas d'acide nucléique permettant l'expression d'une séquence nucléotidique codant le fragment C-terminal de la chaîne α de la protéine C4BP impliquée dans la polymérisation de la protéine C4BP.

3. Procédé selon l'une des revendications 1 ou 2, **caractérisé en ce que** ledit polypeptide hétérologue est choisi dans le groupe constitué des enzymes, des facteurs de régulation d'activité enzymatique, des ligands de récepteurs, des haptènes, des antigènes, des anticorps, des fragments d'anticorps, des médicaments, des récepteurs, notamment des récepteurs monochaînes ou des accepteurs protéiques de médicament.

4. Procédé selon l'une des revendications 1 à 3, **caractérisé en ce que** le fragment 193-252 de la chaîne β de la protéine C4BP humaine a la séquence suivante :

5. Procédé selon l'une des revendications 1 à 3, **caractérisé en ce que** la production de protéines dimériques en utilisant une séquence codant un variant fonctionnel du fragment 193-252 tel que défini à la revendication 1, est au moins 80% égale à celle obtenue avec une séquence native codant le fragment 193-252, dans un système d'expression identique.

6. Procédé selon l'une des revendications 1 à 3, **caractérisé en ce que** le polypeptide de fusion comprend en amont du fragment 193-252, une séquence adjacente de la chaîne β, incluant par exemple au moins un motif SCR et/ou 4 ou 5 acides aminés [GS].

7. Procédé selon la revendication 6, dans lequel lorsque la séquence adjacente code pour les deux premiers motifs SCR de la chaîne β, ces motifs sont mutés par addition, délétion ou substitution d'acides aminés de sorte à supprimer la possibilité d'interaction avec la protéine S.

8. Procédé selon l'une des revendications 1 à 7, **caractérisé en ce que** lesdites cellules hôtes sont choisies parmi des lignées cellulaires eucaryotes, de préférence des cellules d'insectes Sf9.

9. Procédé selon l'une quelconque des revendications 1 à 8, **caractérisé en ce que** le vecteur est un vecteur d'expression recombinant pour baculovirus.

10. Procédé d'obtention d'hétérodimères, ledit procédé comprenant :
a. la transfection de cellules hôtes par un ou plusieurs vecteurs permettant l'expression d'une ou plusieurs séquences nucléotidiques codant :
i. un premier polypeptide de fusion, ledit polypeptide de fusion comprenant le fragment 193-252 de la chaîne β de la protéine C4BP humaine ou un variant fonctionnel de ce fragment tel que défini dans la revendication 1, et un premier polypeptide hétérologue à ladite chaîne β; et
ii. un second polypeptide de fusion, ledit polypeptide de fusion comprenant au moins le fragment 193-252 de la chaîne β de la protéine C4BP humaine ou un variant fonctionnel de ce fragment tel que défini dans la revendication 1, et un second polypeptide hétérologue à ladite chaîne β dont la séquence est différente de celle du premier polypeptide hétérologue ;
b. la culture des cellules transfectées dans des conditions appropriées pour l'expression de la ou des séquences nucléotidiques codant le premier et le second polypeptides de fusion et l'association de deux polypeptides de fusion *in vivo* pour former une protéine hétérodimérique ; et
c. la récupération des protéines hétérodimériques formées.

11. Procédé d'obtention d'hétérodimères selon la revendication 10, dans lequel lesdits premier et second polypeptides hétérologues sont choisis, indépendamment l'un de l'autre, dans le groupe constitué des enzymes, des facteurs de régulation d'activité enzymatique, des ligands de récepteurs, des haptènes, des antigènes, des anticorps, des fragments d'anticorps, des médicaments, des récepteurs, notamment des récepteurs monochaînes ou des accepteurs protéiques de médicament.

12. Procédé d'obtention d'hétérodimères selon l'une quelconque des revendications 10 à 11, lesdits premier et second polypeptides hétérologues des hétérodimères comportant chacun un site anticorps différent de l'autre polypeptide, un ligand différent de l'autre polypeptide ou un récepteur monochaîne différent de l'autre polypeptide.

13. Protéine dimérique recombinante, **caractérisée en ce qu'**elle est constituée de deux polypeptides de fusion, chaque polypeptide de fusion comprenant au moins un fragment constitué des acides aminés des positions 193 à 252 de la chaîne β de la protéine C4BP humaine ou un variant fonctionnel de ce fragment tel que défini dans la revendication 1, ledit polypeptide de fusion comprenant en outre un polypeptide hétérologue à ladite chaîne β.

14. Protéine dimérique recombinante selon la revendication 13, **caractérisée en ce que** le fragment de la chaîne β de la protéine C4BP humaine comprenant au moins les acides aminés des positions 193 à 252, ou un variant fonctionnel de ce fragment tel que défini dans la revendication 1, est fusionné à l'extrémité C-terminale du polypeptide hétérologue.

15. Protéine dimérique selon l'une quelconque des revendications 13 à 14, **caractérisée en ce que** le polypeptide hétérologue est choisi dans le groupe constitué des enzymes, des facteurs de régulation d'activité enzymatique, des ligands de récepteurs, des haptènes, des antigènes, des anticorps, des fragments d'anticorps, des médicaments, des récepteurs, notamment des récepteurs monochaînes ou des accepteurs protéiques de médicament.

16. Protéine dimérique selon l'une quelconque des revendications 13 à 15, **caractérisée en ce que** le polypeptide hétérologue est un fragment d'anticorps comportant tout ou partie des régions variables d'un anticorps fonctionnel pour la liaison Antigène-Anticorps.

17. Protéine hétérodimérique recombinante, **caractérisée en ce qu'**elle est constituée d'un premier et d'un second polypeptides de fusion, chaque polypeptide de fusion comprenant au moins un fragment constitué des acides aminés des positions 193 à 252 de la chaîne β de la protéine C4BP humaine ou un variant fonctionnel de ce fragment tel que défini dans la revendication 1, ledit premier polypeptide de fusion comprenant en outre un polypeptide hétérologue à ladite chaîne β, et le second polypeptide de fusion comprenant un second polypeptide hétérologue à ladite chaîne β différent du premier polypeptide hétérologue.

18. Protéine hétérodimérique recombinante selon la revendication 17, **caractérisée en ce que** le fragment de la chaîne β de la protéine C4BP humaine, comprenant au moins les acides aminés des positions 193 à 252, du premier et du second polypeptides de fusion, ou un variant fonctionnel de ce fragment, sont respectivement fusionnés à l'extrémité C-terminale du premier polypeptide et du second polypeptides hétérologues.

19. Protéine hétérodimérique selon l'une quelconque des revendications 17 à 18, **caractérisée en ce que** le premier et le second polypeptides hétérologues sont choisis, indépendamment l'un de l'autre, dans le groupe constitué des enzymes, des facteurs de régulation d'activité enzymatique, des ligands de récepteurs, des haptènes, des antigènes, des anticorps, des fragments d'anticorps, des médicaments, des récepteurs, notamment des récepteurs monochaînes ou des accepteurs protéiques de médicament.

20. Protéine hétérodimérique selon la revendication 19, **caractérisée en ce que** le fragment d'anticorps comporte tout ou partie des régions variables d'un anticorps fonctionnel pour la liaison Antigène-Anticorps.

21. Protéine hétérodimérique selon l'une quelconque des revendications 17 à 20, **caractérisée en ce que** le premier et le second polypeptides hétérologues sont des ligands différents.

22. Protéine hétérodimérique selon l'une quelconque des revendications 17 à 20, **caractérisée en ce que** le premier et le second polypeptides hétérologues sont des sites anticorps différents.

23. Protéine hétérodimérique selon l'une quelconque des revendications 17 à 20, **caractérisée en ce que** le premier et le second polypeptides hétérologues sont des récepteurs monochaînes différents.

24. Protéine hétérodimérique selon l'une quelconque des revendications 17 à 20 **caractérisée en ce que** le premier polypeptide hétérologue est un anticorps et le second polypeptide hétérologue est un médicament.

25. Protéine hétérodimérique selon l'une quelconque des revendications 17 à 20 **caractérisée en ce que** le premier polypeptide hétérologue est un anticorps et le second polypeptide hétérologue est un accepteur protéique de médicament.

26. Protéine dimérique ou hétérodimérique recombinante selon la revendication 13 à 25, **caractérisée en ce que** lesdits polypeptides de fusion sont associés par liaison covalente entre deux cystéines du fragment de la chaîne β de la protéine C4BP humaine.

27. Cellule eucaryote recombinante permettant la synthèse d'une protéine dimérique ou hétérodimérique selon l'une des revendications 13 à 26, **caractérisée en ce qu'**elle est susceptible d'être obtenue par la mise en oeuvre de l'étape (a) du procédé tel que défini à la revendication 1.

28. Cellule eucaryote selon la revendication 27, **caractérisée en ce qu'**il s'agit d'une cellule d'insecte, de préférence il s'agit d'une lignée cellulaire Sf9.

29. Utilisation, dans un procédé de production d'une protéine dimérique recombinante, d'un acide nucléique codant un polypeptide de fusion, ledit polypeptide de fusion comprenant au moins le fragment constitué des acides aminés des positions 193 à 252 de la chaîne β de la protéine C4BP humaine ou un variant fonctionnel de fragment tel que défini dans la revendication 1, ledit polypeptide de fusion comprenant en outre un polypeptide hétérologue à ladite chaîne β.

30. Utilisation, dans un procédé de production d'une protéine hétérodimérique recombinante,
a) d'un premier acide nucléique codant un premier polypeptide de fusion, ledit premier polypeptide de fusion comprenant au moins le fragment constitué des acides aminés des positions 193 à 252 de la chaîne β de la protéine C4BP humaine ou un variant fonctionnel tel que défini dans la revendication 1, ledit premier polypeptide de fusion comprenant en outre un premier polypeptide hétérologue à ladite chaîne β, et
b) d'un second acide nucléique codant un second polypeptide de fusion, ledit second polypeptide de fusion comprenant au moins le fragment constitué des acides aminés des positions 193 à 252 de la chaîne β de la protéine C4BP humaine ou un variant fonctionnel tel que défini dans la revendication 1, ledit second polypeptide de fusion comprenant en outre un second polypeptide hétérologue à la dite chaîne β dont la séquence est différente de celle du premier polypeptide hétérologue.

## Patentansprüche

1. Verfahren zum Erhalten eines dimeren rekombinanten Proteins, umfassend:
a. Transfektion von Wirtszellen durch einen Vektor, der die Expression einer ein Fusionspolypeptid kodierenden Nukleotidsequenz erlaubt, wobei das Fusionspolypeptid mindestens das aus den Aminosäuren der Positionen 193 bis 252 der β-Kette des humanen C4BP-Proteins bestehende Fragment oder eine funktionelle Variante dieses Fragments umfasst, wobei die funktionelle Variante die Fähigkeit bewahrt, ein mindestens dimeres Protein zu bilden und aus der Gruppe ausgewählt ist bestehend aus:
a1) einer modifizierten Sequenz des Fragments 193-252 der β-Kette, von der weniger als 25% der Aminosäuren des Fragments 193-252, bevorzugt weniger als 10% eliminiert oder ausgetauscht wurden, in der die an Positionen 202 und 216 gelegenen Cysteine sowie mindestens 3 Aminosäuren vor und nach jedem Cystein konserviert wurden;
a2) einer modifizierten Sequenz des Fragments 193-252 der β-Kette, in der ein für die Dimerisierung verantwortliches Cystein durch eine Aminosäure ausgewählt aus Alanin, Valin, Phenylalanin, Prolin, Methionin, Isoleucin, Leucin und Tryptophan substituiert ist, und eine andere Aminosäure des Fragments durch ein Cystein substituiert ist;
a3) einer modifizierten Sequenz des Fragments 193-252 der β-Kette durch Insertion einer zur β-Kette heterologen Sequenz zwischen die für die Dimerisierung verantwortlichen Cysteine; und
a4) einer modifizierten Sequenz des Fragments 193-252 der β-Kette durch Deletion von Aminosäuren zwischen den für die Dimerisierung verantwortlichen Cysteinen;
wobei das Fusionspolypeptid außerdem ein zur β-Kette heterologes Polypeptid umfasst,
b. Kultivierung der transfizierten Zellen unter geeigneten Bedingungen zur Expression der das Fusionspolypeptid kodierenden Nukleotidsequenz und kovalenten Assoziation der zwei Fusionspolypeptide *in vivo,* um ein dimeres Protein zu bilden, und
c. Gewinnen der gebildeten dimeren Proteine.

2. Verfahren nach Anspruch 1, **dadurch gekennzeichnet, dass** die transfizierten Zellen keine Nukleinsäure enthalten, die die Expression einer Nukleotidsequenz erlaubt, die das C-terminale Fragment der α-Kette des C4BP-Proteins kodiert, die in der Polymerisierung des C4BP-Proteins einbezogen ist.

3. Verfahren nach einem der Ansprüche 1 oder 2, **dadurch gekennzeichnet, dass** das heterologe Polypeptid ausgewählt ist aus der Gruppe bestehend aus Enzymen, Faktoren zur Regulierung von enzymatischer Aktivität, Rezeptorliganden, Haptenen, Antigenen, Antikörpern, Antikörperfragmenten, Arzneimitteln, Rezeptoren, insbesondere einkettigen Rezeptoren, oder Arzneimittel-Proteinakzeptoren.

4. Verfahren nach einem der Ansprüche 1 bis 3, **dadurch gekennzeichnet, dass** das Fragment 193-252 der β-Kette des humanen C4BP-Proteins die folgende Sequenz aufweist:

5. Verfahren nach einem der Ansprüche 1 bis 3, **dadurch gekennzeichnet, dass** die Herstellung von dimeren Proteinen unter Verwendung einer Sequenz, die eine funktionelle Variante des Fragments 193-252 wie in Anspruch 1 definiert kodiert, mindestens 80% gleich zu der mit einer nativen, das Fragment 193-252 kodierenden Sequenz in einem identischen Expressionssystem erhaltenen ist.

6. Verfahren nach einem der Ansprüche 1 bis 3, **dadurch gekennzeichnet, dass** das Fusionspolypeptid vor Fragment 193-252 eine an die β-Kette angrenzende Sequenz umfasst, die zum Beispiel mindestens ein SCR-Motif und/oder 4 oder 5 Aminosäuren [GS] einschließt.

7. Verfahren nach Anspruch 6, in dem, wenn die angrenzende Sequenz zwei erste SCR-Motive der β-Kette kodiert, diese Motive durch Addition, Deletion oder Substitution von Aminosäuren mutiert werden, um die Möglichkeit der Wechselwirkung mit Protein S zu beseitigen.

8. Verfahren nach einem der Ansprüche 1 bis 7, **dadurch gekennzeichnet, dass** die Wirtszellen aus eukaryotischen Zellinien ausgewählt sind, bevorzugt Sf9-Insektenzellen.

9. Verfahren nach einem der Ansprüche 1 bis 8, **dadurch gekennzeichnet, dass** der Vektor ein rekombinanter Expressionvektor für Baculovirus ist.

10. Verfahren zum Erhalten von Heterodimeren, wobei das Verfahren umfasst:
a. Transfektion von Wirtszellen durch einen oder mehrere Vektoren, die die Expression von einer oder mehreren Nukleotidsequenzen erlauben, die kodieren:
i. ein erstes Fusionspolypeptid, wobei das Fusionspolypeptid das Fragment 193-252 der β-Kette des humanen C4BP-Proteins oder eine funktionelle Variante des wie in Anspruch 1 definierten Fragments, und ein erstes zu dieser β-Kette heterologes Polypeptid umfasst; und
ii. ein zweites Fusionspolypeptid, wobei das Fusionspolypeptid mindestens das Fragment 193-252 der β-Kette des humanen C4BP-Proteins oder eine funktionelle Variante des wie in Anspruch 1 definierten Fragments und ein zweites zur β-Kette heterologes Polypeptid, dessen Sequenz zu der des ersten heterologen Polypeptids verschieden ist, umfasst;
b. Kultivierung der transfizierten Zellen unter geeigneten Bedingungen zur Expression der Nukleotidsequenz oder der Nukleotidsequenzen, die das erste und das zweite Fusionspolypeptid kodiert/kodieren, und Assoziation der zwei Fusionspolypeptide *in vivo,* um ein heterodimeres Protein zu bilden; und
c. Gewinnen der gebildeten heterodimeren Proteine.

11. Verfahren zum Erhalten von Heterodimeren nach Anspruch 10, in dem die ersten und zweiten heterologen Polypeptide unabhängig voneinander ausgewählt sind aus der Gruppe bestehend aus Enzymen, Faktoren zur Regulierung von enzymatischer Aktivität, Rezeptorliganden, Haptenen, Antigenen, Antikörpern, Antikörperfragmenten, Arzneimitteln, Rezeptoren, insbesondere einkettigen Rezeptoren, oder Arzneimittel-Proteinakzeptoren.

12. Verfahren zum Erhalten von Heterodimeren nach einem beliebigen der Ansprüche 10 bis 11, wobei die ersten und zweiten heterologen Polypeptide der Heterodimere jedes eine zum anderen Polyeptid verschiedene Antikörperstelle, einen zum anderen Polypeptid verschiedenen Liganden, oder einen zum anderen Polypeptid verschiedenen einkettigen Rezeptor enthalten.

13. Rekombinantes dimeres Protein, **dadurch gekennzeichnet, dass** es aus zwei Fusionspolypeptiden besteht, wobei jedes Fusionspolypeptid mindestens ein Fragment umfasst, das aus Aminosäuren der Positionen 193 bis 252 der β-Kette des humanen C4BP-Proteins oder einer funktionellen Variante dieses Fragments wie in Anspruch 1 definiert besteht, wobei das Fusionspolypeptid außerdem ein anderes zur β-Kette heterologes Polypeptid umfasst.

14. Rekombinantes dimeres Protein nach Anspruch 13, **dadurch gekennzeichnet, dass** das Fragment der β-Kette des humanen C4BP-Proteins, das mindestens die Aminosäuren der Positionen 193 bis 252 umfasst, oder eine funktionelle Variante dieses Fragments wie in Anspruch 1 definiert, am C-terminalen Ende des heterologen Polypeptids fusioniert ist.

15. Dimeres Protein nach einem beliebigen der Ansprüche 13 bis 14, **dadurch gekennzeichnet, dass** das heterologe Polypeptid ausgewählt ist aus der Gruppe bestehend aus Enzymen, Faktoren zur Regulierung von enzymatischer Aktivität, Rezeptorliganden, Haptenen, Antigenen, Antikörpern, Antikörperfragmenten, Arzneimitteln, Rezeptoren, insbesondere einkettigen Rezeptoren, oder Arzneimittel-Proteinakzeptoren.

16. Dimeres Protein nach einem beliebigen der Ansprüche 13 bis 15, **dadurch gekennzeichnet, dass** das heterologe Polypeptid ein Antikörperfragment ist, das alles oder einen Teil der variablen Regionen eines funktionellen Antikörpers für die Antigen-Antikörper-Bindung enthält.

17. Rekombinantes heterodimeres Protein, **dadurch gekennzeichnet, dass** es aus einem ersten und einem zweiten Fusionspolypeptid besteht, wobei jedes Fusionspolypeptid mindestens ein Fragment umfasst, das aus den Aminosäuren der Positionen 193 bis 252 der β-Kette des humanen C4BP-Proteins oder einer funktionellen Variante dieses Fragments wie in Anspruch 1 definiert besteht, wobei das erste Fusionspolypeptid außerdem ein zur β-Kette heterologes Polypeptid umfasst, und das zweite Fusionspolypeptid ein zweites zur β-Kette heterologes vom ersten heterologen Polypeptid verschiedenes Polypeptid umfasst.

18. Rekombinantes heterodimeres Protein nach Anspruch 17, **dadurch gekennzeichnet, dass** das Fragment der β-Kette des humanen C4BP-Proteins, das mindestens die Aminosäuren der Positionen 193 bis 252 umfasst, des ersten und des zweiten Fusionspolypeptids, oder eine funktionelle Variante dieses Fragments, jeweils am C-terminalen Ende des ersten Polypeptids und des zweiten heterologen Polypeptids fusioniert sind.

19. Heterodimeres Protein nach einem beliebigen der Ansprüche 17 bis 18, **dadurch gekennzeichnet, dass** das erste und das zweite heterologe Polypeptid unabhängig voneinander ausgewählt sind aus der Gruppe bestehend aus Enzymen, Faktoren zur Regulierung von enzymatischer Aktivität, Rezeptorliganden, Haptenen, Antigenen, Antikörpern, Antikörperfragmenten, Arzneimitteln, Rezeptoren, insbesondere einkettigen Rezeptoren, oder Arzneimittel-Proteinakzeptoren.

20. Heterodimeres Protein nach Anspruch 19, **dadurch gekennzeichnet, dass** das Antikörperfragment alles oder einen Teil der variablen Regionen eines funktionellen Antikörpers für die Antigen-Antikörper-Bindung enthält.

21. Heterodimeres Protein nach einem beliebigen der Ansprüche 17 bis 20, **dadurch gekennzeichnet, dass** das erste und das zweite heterologe Polypeptid verschiedene Liganden sind.

22. Heterodimeres Protein nach einem beliebigen der Ansprüche 17 bis 20, **dadurch gekennzeichnet, dass** das erste und das zweite heterologe Polypeptid verschiedene Antikörperstellen sind.

23. Heterodimeres Protein nach einem beliebigen der Ansprüche 17 bis 20, **dadurch gekennzeichnet, dass** das erste und das zweite heterologe Polypeptid verschiedene einkettige Rezeptoren sind.

24. Heterodimeres Protein nach einem beliebigen der Ansprüche 17 bis 20, **dadurch gekennzeichnet, dass** das erste heterologe Polypeptid ein Antikörper ist und das zweite heterologe Polypeptid ein Arzneimittel ist.

25. Heterodimeres Protein nach einem beliebigen der Ansprüche 17 bis 20, **dadurch gekennzeichnet, dass** das erste heterologe Polypeptid ein Antikörper ist und das zweite heterologe Polypeptid ein Arzneimittel-Proteinakzeptor ist.

26. Dimeres oder heterodimeres Protein nach Anspruch 13 bis 25, **dadurch gekennzeichnet, dass** die Fusionspolypeptide durch kovalente Bindung zwischen zwei Cysteinen des Fragments der β-Kette des humanen C4BP-Proteins assoziiert sind.

27. Rekombinante Eukaryotenzelle, die die Synthese eines dimeren oder heterodimeren Proteins gemäß einem der Ansprüche 13 bis 26 erlaubt, **dadurch gekennzeichnet, dass** sie fähig ist, durch das Ausführen des Schritts (a) des wie in Anspruch 1 definierten Verfahrens erhalten zu werden.

28. Eukaryotenzelle nach Anspruch 27, **dadurch gekennzeichnet, dass** es sich um eine Insektenzelle handelt, bevorzugt es sich um eine Sf9-Zelllinie handelt.

29. Verwendung, in einem Verfahren zur Herstellung eines dimeren rekombinanten Proteins, einer ein Fusionspolypeptid kodierenden Nukleinsäure, wobei das Fusionspolypeptid mindestens das aus den Aminosäuren der Positionen 193 bis 252 bestehende Fragment der β-Kette des humanen C4BP-Proteins oder eine funktionelle Variante des Fragments wie in Anspruch 1 definiert umfasst, wobei das Fusionspolypeptid außerdem ein zur β-Kette heterologes Polypeptid umfasst.

30. Verwendung in einem Verfahren zur Herstellung eines heterodimeren rekombinanten Proteins,
a. einer ersten Nukleinsäure, die ein erstes Fusionspolypeptid kodiert, wobei das erste Fusionspolypeptid mindestens das aus den Aminosäuren der Positionen 193 bis 252 bestehende Fragment der β-Kette des humanen C4BP-Proteins oder eine funktionelle Variante des Fragments wie in Anspruch 1 definiert umfasst, wobei das erste Fusionspolypeptid außerdem ein erstes zur β-Kette heterologes Polypeptid umfasst, und
b. einer zweiten Nukleinsäure, die ein zweites Fusionspolypeptid kodiert, wobei das zweite Fusionspolypeptid mindestens das aus den Aminosäuren der Positionen 193 bis 252 bestehende Fragment der β-Kette des humanen C4BP-Proteins oder eine funktionelle Variante des Fragments wie in Anspruch 1 definiert umfasst, wobei das zweite Fusionspolypeptid außerdem ein zweites zur β-Kette heterologes Polypeptid umfasst, dessen Sequenz verschieden zu der des ersten heterologen Polypeptids ist.

## Claims

1. A method for producing a recombinant dimeric protein, comprising:
a) transfecting host cells with a vector enabling expression of a nucleotide sequence coding for a fusion polypeptide, said fusion polypeptide comprising at least the fragment constituted by amino acids in positions 193 to 252 of the β chain of the human C4BP protein or a functional variant of said fragment, said functional variant conserving the capacity to form an at least dimeric protein and being selected from the group constituted by:
a1) a modified sequence of the 193 - 252 fragment of the P chain, wherein less than 25% of the amino acids of the 193-252 fragment, preferably less than 10%, have been cut out or replaced, in which the cysteines located in positions 202 and 216 as well as at least 3 amino acids upstream and downstream of each cysteine have been conserved;
a2) a modified sequence of the 193 - 252 fragment of the P chain, in which a cysteine responsible for dimerization is substituted with an amino acid selected from alanine, valine, phenylalanine, proline, methionine, isoleucine, leucine and tryptophan, and another amino acid of the fragment is substituted with a cysteine;
a3) a sequence of the 193 - 252 fragment of the P chain modified by insertion of a sequence which is heterologous to the P chain, between the cysteines responsible for dimerization; and
a4) a sequence of the 193 - 252 fragment of the P chain modified by cutting out amino acids between the cysteines responsible for dimerization;
said fusion polypeptide further comprising a polypeptide which is heterologous to said P chain,
b) culturing transfected cells under conditions which are suitable for expressing the nucleotide sequence coding for the fusion polypeptide and covalently associating two fusion polypeptides *in vivo* to form a dimeric protein; and
c) recovering the dimeric proteins formed.

2. A method according to claim 1, **characterized in that** the transfected cells contain no nucleic acid enabling expression of a nucleotide sequence coding for the C-terminal fragment of the α chain of the C4BP protein involved in polymerization of the C4BP protein.

3. A method according to claim 1 or claim 2, **characterized in that** said heterologous polypeptide is selected from the group constituted by enzymes, enzymatic activity regulation factors, receptor ligands, haptens, antigens, antibodies, antibody fragments, drugs and receptors, in particular monochain receptors or proteic drug acceptors.

4. A method according to one of claims 1 to 3, **characterized in that** the 193 - 252 fragment of the β chain of the human C4BP protein has the following sequence: LIQEAPKPECEKALLAFQESKNLCEAMENFMQQLKESGMTMEELKYSLEL KKAELKAKLL.

5. A method according to one of claims 1 to 3, **characterized in that** the production of dimeric proteins using a sequence coding for a functional variant of the 193 - 252 fragment of the β chain as defined in claim 1 is at least 80% equal to that obtained with a native sequence coding for the 193 - 252 fragment in an identical expression system.

6. A method according to one of claims 1 to 3, **characterized in that** the fusion polypeptide comprises, upstream of the 193-252 fragment, a sequence adjacent to the P chain including, for example, at least one SCR motif and/or 4 or 5 [GS] amino acids.

7. A method according to claim 6 in which, when the adjacent sequence codes for the first two SCR motifs of the P chain, these motifs are mutated by addition, deletion or substitution of amino acids in a manner such as to cut out the possibility of interaction with the S protein.

8. A method according to one of claims 1 to 7, **characterized in that** said host cells are selected from eukaryotic cell lines, preferably sf9 insect cells.

9. A method according to any one of claims 1 to 8, **characterized in that** the vector is a recombinant expression vector for baculovirus.

10. A method for producing heterodimers, said method comprising:
a. transfecting host cells with one or more vectors to enable the expression of one or more nucleotide sequences coding for:
i. a first fusion polypeptide, said fusion polypeptide comprising the 193 - 252 fragment of the β chain of the human C4BP protein or a functional variant of said fragment as defined in claim 1, and a first polypeptide which is heterologous to said β chain; and
ii. a second fusion polypeptide, said fusion polypeptide comprising at least the 193 - 252 fragment of the β chain of the human C4BP protein or a functional variant of said fragment as defined in claim 1, and a second polypeptide which is heterologous to said β chain the sequence of which is different from that of the first heterologous polypeptide;
b. culturing transfected cells under conditions appropriate for expressing the nucleotide sequence or sequences coding for the first and second fusion polypeptides and associating two fusion polypeptides *in vivo* in order to form a heterodimeric protein; and
c. recovering the heterodimeric proteins formed.

11. A method for producing heterodimers according to claim 10, in which said first and second heterologous polypeptides are selected, independently of each other, from the group constituted by enzymes, enzymatic activity regulation factors, receptor ligands, haptens, antigens, antibodies, antibody fragments, drugs and receptors, in particular monochain receptors or proteic drug acceptors.

12. A method for producing heterodimers according to claim 10 or claim 11, said first and second heterologous polypeptides of the heterodimers each comprising an antibody site which differs from the other polypeptide, a ligand which differs from the other polypeptide or a monochain receptor which differs from the other polypeptide.

13. A recombinant dimeric protein, **characterized in that** it is constituted by two fusion polypeptides, each fusion polypeptide comprising at least a fragment constituted by amino acids in positions 193 to 252 of the β chain of the human C4BP protein or a functional variant of said fragment as defined in claim 1, said fusion polypeptide further comprising a polypeptide which is heterologous to said β chain.

14. A recombinant dimeric protein according to claim 13, **characterized in that** the fragment of the β chain of the human C4BP protein comprising at least the amino acids in positions 193 to 252 or a functional variant of said fragment as defined in claim 1 is fused to the C-terminal end of the heterologous polypeptide.

15. A dimeric protein according to claim 13 or claim 14, **characterized in that** the heterologous polypeptide is selected from the group constituted by enzymes, enzymatic activity regulating factors, receptor ligands, haptens, antigens, antibodies, antibody fragments, drugs and receptors, especially monochain receptors or proteic drug acceptors.

16. A dimeric protein according to any one of claims 13 to 15, **characterized in that** the heterologous polypeptide is an antibody fragment comprising all or part of the variable regions of an antibody which is functional for antigen-antibody binding.

17. A recombinant heterodimeric protein, **characterized in that** it is constituted by a first and a second fusion polypeptide, each fusion polypeptide comprising at least one fragment constituted by amino acids in positions 193 to 252 of the β chain of the human C4BP protein or a functional variant of said fragment as defined in claim 1, said first fusion polypeptide further comprising a polypeptide which is heterologous to said β chain, and the second fusion polypeptide comprising a second polypeptide which is heterologous to said β chain which differs from the first heterologous polypeptide.

18. A recombinant heterodimeric protein according to claim 17, **characterized in that** the fragment of the β chain of the human C4BP protein comprising at least the amino acids in positions 193 to 252 of the first and second fusion polypeptide, or a functional variant of said fragment, are respectively fused to the C-terminal end of the first polypeptide and the second heterologous polypeptides.

19. A heterodimeric protein according to claim 17 or claim 18, **characterized in that** the first and second heterologous polypeptides are selected, independently of each other, from the group constituted by enzymes, enzymatic activity regulating factors, receptor ligands, haptens, antigens, antibodies, antibody fragments, drugs and receptors, especially monochain receptors or proteic drug acceptors.

20. A heterodimeric protein according to claim 19, **characterized in that** the antibody fragment comprises all or part of the variable regions of an antibody which is functional for antigen-antibody binding.

21. A heterodimeric protein according to any one of claims 17 to 20, **characterized in that** the first and second heterologous polypeptides are different ligands.

22. A heterodimeric protein according to any one of claims 17 to 20, **characterized in that** the first and second heterologous polypeptides are different antibody sites.

23. A heterodimeric protein according to any one of claims 17 to 20, **characterized in that** the first and second heterologous polypeptides are different monochain receptors.

24. A heterodimeric protein according to any one of claims 17 to 20, **characterized in that** the first heterologous polypeptide is an antibody and the second heterologous polypeptide is a drug.

25. A heterodimeric protein according to any one of claims 17 to 20, **characterized in that** the first heterologous polypeptide is an antibody and the second heterologous polypeptide is a proteic drug acceptor.

26. A recombinant heterodimeric or dimeric protein according to claims 13 to 25, **characterized in that** said fusion polypeptides are associated by covalent bonding between two cysteines of the β chain fragment of the human C4BP protein.

27. A recombinant eukaryotic cell enabling synthesis of a dimeric or heterodimeric protein according to one of claims 13 to 26, **characterized in that** it is capable of being obtained by carrying out step a) of the method defined in claim 1.

28. A eukaryotic cell according to claim 27, **characterized in that** it is an insect cell, preferably a sf9 cell line.

29. Use, in a method for producing a recombinant dimeric protein, of a nucleic acid coding for a fusion polypeptide, said fusion polypeptide comprising at least the fragment constituted by amino acids in positions 193 to 252 of the β chain of the human C4BP protein or a functional variant of the fragment as defined in claim 1, said fusion polypeptide further comprising a polypeptide which is heterologous to said β chain.

30. Use, in a method for producing a recombinant heterodimeric protein, of:
a) a first nucleic acid coding for a first fusion polypeptide, said first fusion polypeptide comprising at least the fragment constituted by amino acids in positions 193 to 252 of the β chain of the human C4BP protein or a functional variant as defined in claim 1, said first fusion polypeptide further comprising a first polypeptide which is heterologous to said β chain; and
b) a second nucleic acid coding for a second fusion polypeptide, said second fusion polypeptide comprising at least the fragment constituted by amino acids in positions 193 to 252 of the β chain of the human C4BP protein or a functional variant as defined in claim 1, said second fusion polypeptide further comprising a second polypeptide which is heterologous to said β chain the sequence of which differs from that of the first heterologous polypeptide.


### SEQUENCE LISTING

<110> UNIVERSITE DE REIMS CHAMPAGNE-ARDENNE
<120> UTILISATION DU GENE CODANT LA CHAINE BETA DE LA,PROTEINE C4BP DANS LA
   PRODUCTION DE PROTEINES DIMERIQUES RECOMBINANTES
<130> B5964A - AD/LV/SDU
<140> PCT/FRXX/XXXXX
   <141> 2005-04-20
<150> FR 0404295
   <151> 2004-04-22
<160> 1
<170> PatentIn version 3.1
<210> 1
   <211> 60
   <212> PRT
   <213> Homo sapiens
<400> 1
   Leu Ile Gln Glu Ala Pro Lys Pro Glu Cys Glu Lys Ala Leu Leu Ala
   1               5                   10                  15
   Phe Gln Glu Ser Lys Asn Leu Cys Glu Ala Met Glu Asn Phe Met Gln
               20                  25                  30
   Gln Leu Lys Glu Ser Gly Met Thr Met Glu Glu Leu Lys Tyr Ser Leu
           35                  40                  45
   Glu Leu Lys Lys Ala Glu Leu Lys Ala Lys Leu Leu
       50                  55                  60